# EUROPEAN PATENT SPECIFICATION

(11) **EP 2 139 482 B1**
(45) Date of publication and mention of the grant of the patent: **16.11.2011**
(21) Application number: 07846860.0
(22) Date of filing: 27.11.2007
(51) Int. Cl.: A61K 31/496, A61P 35/00

(54) **TREATMENT FOR MULTIPLE MYELOMA**
BEHANDLUNG VON MULTIPLEM MYELOM
TRAITEMENT DU MYÉLOME MULTIPLE

(30) Priority: 27.11.2006 US 861120 P; 28.02.2007 US 904138 P
(43) Date of publication of application: 06.01.2010
(73) Proprietor: Ares Trading S.A., 1170 Aubonne (CH)
(72) Inventor: JOURDAN, Michel, 34980 Saint-Gely-Fesc (FR); DREANO, Michel, 74160 Collonges-sous-Salève (FR); KLEIN, Bernard, 34980 Saint Clément de Rivière (FR)
(74) Representative: Merck Serono SA - Geneva Intellectual Property
(86) International application number: PCT/EP2007/010299
(87) International publication number: WO 2008/064866

(56) References cited:
- WO-A-02/46170

## Description

### RELATED APPLICATIONS

This application claims the benefit of U.S. Provisional Application No. 60/861,120, filed November 27, 2006 and U.S. Provisional Application No. 60/904,138, filed February 28, 2007. .

### BACKGROUND OF THE INVENTION

Multiple myeloma is a cancer of the plasma cells. Plasma cells are immune system cells in bone marrow that produce antibodies for fighting diseases and infections. During development, genetic abnormalities can occur that yield malignant plasma cells or multiple myeloma cells. These cells travel through the bloodstream and collect in bone marrow where they cause permanent damage to healthy tissue. Because many organs can be affected by multiple myeloma, the symptoms and signs are variable. Common symptoms include bone lesions, elevated calcium levels, renal failure, anemia, and impaired immune capabilities. Bone damage is caused by rapid proliferation of myeloma cells and release of IL-6, known as osteoclast activating factor, which stimulates osteoclasts to break down bone. The breakdown of bone can also cause the level of calcium in the bloodstream to rise, a condition called hypercalcemia. As myeloma cells crowd out normal cells in the bone marrow, the production of normal blood cells is also impaired. A reduction in the number of white blood cells can increase the risk of infection, whereas decreased red blood cell production can result in anemia. A reduction in platelets can prevent normal blood clotting. In addition, excess M protein and light chain protein produced by the myeloma cells can thicken the blood. These proteins can also damage the kidneys and impair their function. Circulatory problems in the kidneys may occur when myeloma cells thicken the blood. In addition, hypercalcemia overworks the kidneys, resulting in reduced calcium excretion, increased urine production, and the potential for dehydration.

Multiple myeloma is the second most prevalent blood cancer after non-Hodgkin's lymphoma. It represents approximately 1% of all cancer and 2% of all cancer deaths. As to date, there is no treatment that results in complete and lasting recovery from the disease. Therefore, there is a need for new treatment methods for multiple myeloma.

### SUMMARY OF THE INVENTION

The present invention relates to a method of treating multiple myeloma in a subject in need thereof. The method comprises administering to the subject an effective amount of a compound according to Formula I, or a pharmaceutically acceptable salt thereof. The multiple myeloma being treated can be in any of the stages, categories and disease status described below.

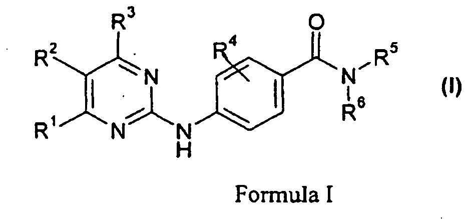

The present invention is based on at least in part the discovery that compounds according to Formula I can inhibit the proliferation of a number of myeloma cell lines (Example 1). These compounds may also exhibited inhibitory effect on the survival of primary myeloma cells without affecting the survival of other bone marrow cells when bone marrow cells of myeloma patients were treated with such compounds (Example 4). When used in combination with Velcade or melphalan, these compounds showed additive effect on inhibition of the proliferation of myeloma cells (Example 3).

### BRIEF DESCRIPTION OF THE DRAWINGS

Compound **A** as used in the Examples and Figures is 1-(4-{4-[4-(4-Chlorophenyl)-pyrimidin-2-ylamino]-benzoyl}-piperazin-1-yl)-ethanone. 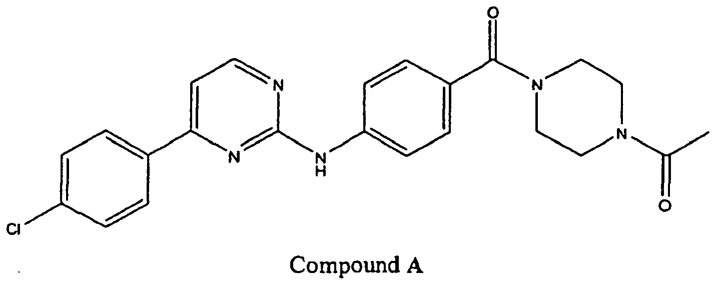
FIG. 1 is a bar graph showing inhibitory effects of compound **A** (IC₅₀ in µM for Figure 1A and IC₉₀ in µM for Figure 1B) on the proliferation of 14 myeloma cell lines.
FIG. 2 is a plot showing time-dependent induction of apoptosis of XG-12 cells treated by compound **A**.
FIG. 3 is a plot showing the inhibitory effects of increasing concentrations of compound **A** on the proliferation of XG-3 cells with or without Velcade at a concentration inducing 10% (IC₁₀) or 50% (IC₅₀) inhibition of XG-3 cell proliferation.
FIG. 4 is a plot showing the inhibitory effects of increasing concentrations of compound **A** on the proliferation of XG-12 cells with or without Melphalan at a concentration inducing 10% (IC₁₀) or 50% (IC₅₀) inhibition of XG-12 cell proliferation.
FIG. 5 is bar graph showing the effect of compound **A** on *ex vivo* survival of CD138⁺ primary myeloma cells from 5 patients with newly diagnosed multiple myeloma.
FIG. 6 is bar graph showing the effect of compound **A** on *ex vivo* survival of non-myeloma bone marrow cells from 5 patients with newly diagnosed multiple myeloma.
FIG. 7 is bar graph showing the effect of compound **A** on *ex vivo* survival of CD34⁺ hematopoietic precursor cells from 5 patients with newly diagnosed multiple myeloma.
FIG. 8 is bar graph showing the effect of compound **A** on *ex vivo* survival of CD138⁺ primary myeloma cells from 5 patients with relapsing multiple myeloma.
FIG. 9 is bar graph showing the effect of compound **A** on *ex vivo* survival of non-myeloma bone marrow cells from 5 patients with relapsing multiple myeloma.
FIG. 10 is bar graph showing the effect of compound **A** on *ex vivo* survival of CD34⁺ hematopoietic precursor cells from 5 patients with relapsing multiple myeloma.

### DETAILED DESCRIPTION OF THE INVENTION

"Aryl" refers to an unsaturated aromatic carbocyclic group of from 6 to 14 carbon atoms having a single ring (e.g. phenyl) or multiple condensed rings (e.g. naphthyl). Preferred aryls include phenyl, naphthyl, phenantrenyl and the like.

"Alkylaryl" refers to an alkyl having at least one alkyl hydrogen atom replaced with an aryl moiety, such as benzyl, -(CH₂)₂phenyl, -(CH₂)₃phenyl, - CH(phenyl)₂, and the like.

"Alkyl" refers to a straight chain or branched, saturated or unsaturated alkyl, cyclic or non-cyclic hydrocarbon having from 1 to 10 carbon atoms, while "lower alkyl" or "C₁-C₆-alkyl" has the same meaning, but only has from 1 to 6 carbon atoms. Representative saturated straight chain alkyls include methyl, ethyl, n-propyl, n-butyl, n-pentyl, n-hexyl, and the like; while saturated branched alkyls include isopropyl, sec-butyl, isobutyl, tert-butyl, isopentyl, and the like. Unsaturated alkyls contain at least one double or triple bond between adjacent carbon atoms (also referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3-methyl-1- butenyl, 2-methyl-2-butenyl, 2,3-dimethyl, 2-butenyl; and the like; while representative straight chain and branched alkynyls include acetylenyl, propynyl, 1-butynyl, 2-butynyl, 1-pentynyi, 2-pentynyl, 3- methyl-1-butynyl, and the like. Representative saturated "cyclic alkyls" include cyclopropyl, cyclobutyl, cyclopentyl, cyclohexyl, and the like; while unsaturated cyclic alkyls include cyclopentenyl and cyclohexenyl, and the like. Cycloalkyls are also referred to herein as "carbocyclic" rings systems, and include bi- and tri-cyclic ring systems having from 8 to 14 carbon atoms, such as a cycloalkyl (such as cyclopentane or cyclohexane) fused to one or more aromatic (such as phenyl) or non-aromatic (such as cyclohexane) carbocyclic rings.

"Alkoxy" refers to -O-(alkyl) or -O-aryl), such as methoxy, ethoxy, n-propyloxy, iso propyloxy, n-butyloxy, iso-butyloxy, phenoxy and the like.

"C₂-C₆-alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (-CH₂CH=CH₂) and the like.

"C₂-C₆-alkynyl" refers to alkynyl groups preferably having from 2 to 6 carbon atoms and having at least 1-2 sites of alkynyl unsaturation, preferred alkynyl groups include ethynyl (-C≡CH), propargyl (-CH₂C≡CH), and the like.

"Halogen" refers to fluorine, chlorine, bromine or iodine.

"Keto" refers to a carbonyl group (i. e.,C =O)

"Heteroaryl" refers to an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, and quinazolinyl.

"Heteroalkylaryl" refers to an alkyl having at least one alkyl hydrogen atom replaced with a heteroaryl moiety, such as -CH₂-pyridinyl, -CH₂-pyrimidinyl, and the like.

"Heterocycloalkyl" or "heterocycle"refers to a heterocyclic ring containing from 5 to 10 ring atoms. Specifically, a 5- to 7-membered monocyclic, or 7- to 10-membered bicyclic, heterocyclic ring which is either saturated, unsaturated, or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined above. Thus, in addition to the heteroaryls listed above, heterocycles also include morpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyrindinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

"Alkylheterocycloalkyl" refers to an alkyl having at least one alkyl hydrogen atom replaced with a heterocycle, such as 2-(1-pyrrolidinyl)ethyl, 4-morpholinylmethyl, (1-methyl-4-piperidinyl)methyl and the like.

The term "substituted" as used herein refers to any of the above groups (i. e. alkyl, aryl, alkyl aryl, heterocyclyl and heterocycloalkyl) wherein at least one hydrogen atom is replaced with a substituent. In the case of a keto substituent ("C(=O)") two hydrogen atoms are replaced. Substituents include halogen, hydroxy, alkyl, substituted alkyl (such as haloalkyl, mono- or all-substituted aminoalkyl, alkyloxyalkyl, and the like), aryl, substituted aryl, arylalkyl, substituted arylalkyl, heterocycloalkyl, substituted heterocycloalkyl, alkylheterocycloalkyl, substituted alkylheterocycloalkyl, -NRₐR_{b}, -NRₐC(=O)R_{b}, -NRₐC(=O)NRₐR_{b}, -NRₐC(=O)OR_{b} - NRₐSO₂R_{b}, -ORₐ, -C(=O)Rₐ, -C(=O)ORₐ, -C(=O)NRₐR_{b}, -OC(=O)Rₐ, -OC(=O)ORₐ, -OC(=O)NRₐR_{b}, -NRₐSO₂R_{b}, or a radical of the formula Y-Z-Rₐ where Y is alkanediyl, substituted alkanediyl, or a direct bond, Z is -O-, -S-, S(=O)-, -S(=O)₂-, - N(R_{b})-, -C(=O)-, -C(=O)O-, -OC(=O)-, -N(R_{b})C(=O)-, -C(=O)N(R_{b})- or a direct bond, wherein Rₐ and R_{b} are the same or different and independently hydrogen, amino, alkyl, substituted alkyl (including halogenated alkyl), aryl, substituted aryl, alkylaryl, substituted alkylaryl, heterocycloalkyl, substituted heterocycloalkyl, alkylheterocyloalkyl or substituted alkylheterocycloalkyl, or wherein Rₐ and R_{b} taken together with the nitrogen atom to which they are attached form a heterocycle or substituted heterocycle.

"Haloalkyl" refers to an alkyl having one or more hydrogen atoms replaced with halogen, such as -CF₃.

"Hydroxyalkyl" means alkyl having one or more hydrogen atoms replaced with hydroxy, such as -CH₂OH.

"Sulfonyl" refers to group "-SO₂-R" wherein R is selected from H, aryl, heteroaryl, C₁-C₆-alkyl, C₁-C₆-alkyl substituted with halogens, (*e.g*., an -SO₂-CF₃ group), C₂-C₆-alkenyl, C₂-C₆-alkynyl, C₃-C₈-cycloalkyl, heterocycloalkyl, aryl, heteroaryl, C₁-C₆-alkyl aryl, C₁C₆-alkyl heteroaryl, C₂-C₆-alkenyl aryl, C₂-C₆-alkenyl heteroaryl, C₂-C₆-alkynyl aryl, C₂-C₆-alkynylheteroaryl, C₁-C₆-alkyl cycloalkyl, or C₁-C₆-alkyl heterocycloalkyl.

"Sulfinyl" refers to a group "-S(O)-R" wherein R is selected from H, C₁-C₆-alkyl, C₁-C₆-alkyl substituted with halogens, (*e.g*., an -SO₂-CF₃ group), C₂-C₆-alkenyl, C₂-C₆-alkynyl, C₃-C₈-cycloalkyl, heterocycloalkyl, aryl, heteroaryl, C₁-C₆-alkyl aryl, C₁-C₆-alkyl heteroaryl, C₂-C₆-alkenyl aryl, C₂-C₆-alkenyl heteroaryl, C₂-C₆-alkynyl aryl, C₂-C₆-alkynylheteroaryl, C₁-C₆-alkyl cycloalkyl, or C₁-C₆-alkyl heterocycloalkyl.

"Sulfanyl" refers to groups "-S-R" where R is selected from H, C₁-C₆-alkyl, C₁-C₆-alkyl substituted with halogens, (*e.g*., an -SO₂-CF₃ group), C₂-C₆-alkenyl, C₂-C₆-alkynyl, C₃-C₈-cycloalkyl, heterocycloalkyl, aryl, heteroaryl, C₁-C₆-alkyl aryl, C₁-C₆-alkyl heteroaryl, C₂-C₆-alkenyl aryl, C₂-C₆-alkenyl heteroaryl, C₂-C₆-alkynyl aryl, C₂-C₆-alkynylheteroaryl, C₁-C₆-alkyl cycloalkyl, or C₁-C₆-alkyl heterocycloalkyl.. Preferred sulfanyl groups include methylsulfanyl, ethylsulfanyl, and the like.

"Carboxyl" refers -COOH.

"Amino" refers to the group -NRR' where each R, R' is independently hydrogen or C₁-C₆-alkyl, aryl, heteroaryl, C₁-C₆-alkyl aryl, C₁-C₆-alkyl heteroaryl, cycloalkyl, or heterocycloalkyl, and where R and R', together with the nitrogen atom to which they are attached, can optionally form a 3-8-membered heterocycloalkyl ring.

"Ammonium" refers to a positively charged group -N⁺RR'R", where each R, R', R" is independently C₁-C₆-alkyl, C₁-C₆-alkyl aryl, C₁-C₆-alkyl heteroaryl, cycloalkyl, or heterocycloalkyl, and where R and R', together with the nitrogen atom to which they are attached, can optionally form a 3-8-membered heterocycloalkyl ring.

"HCl" means the hydrochloride salt of compounds depicted by their chemical structure.

"Nitrogen-containing non-aromatic heterocycle" means morpholinyl, thiomorpholinyl, pyrrolidinonyl, pyrrolidinyl, piperidinyl, homopiperidinyl, piperazinyl, homopiperazinyl, hydantoinyl, tetrahydropyrindinyl, tetrahydropyrimidinyl, oxazolidinyl, thiazolidinyl, indolinyl, isoindolinyl, tetrahydroquinolinyl, tetrahydroisoquinolinyl and the like.

"Pharmaceutically acceptable salts or complexes" refer to salts or complexes of the compounds disclosed herein. Examples of such salts include, but are not limited to, salts which are formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, methane sulfonic acid, naphthalene disulfonic acid, and poly-galacturonic acid, as well as salts formed with basic amino acids such as lysine or arginine.

Additionally, salts of compounds containing a carboxylic acid or other acidic functional group(s) can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, N-benzyl-β-phenethylamine, dehydroabietylamine, N,N'-bisdehydroabietylamine, glucamine, N-methylglucamine, collidine, quinine, quinoline, and basic amino acid such as lysine and arginine.

The present invention is directed to a method of treating a subject with multiple myeloma comprising administering to the subject an effective amount of a compound according to Formula I, or a pharmaceutically acceptable salt thereof.

Multiple myeloma is a cancer of plasma cells. The stage of multiple myeloma can be determined by using the International Staging System (ISS). The ISS is based on the assessment of two blood test results, β₂-microglobulin (β₂-M) and albumin, which together showed the greatest prognostic power for multiple myeloma among a number of factors tested. The criteria for determining different stages according to the International Staging System for myeloma is listed below:
- Stage I: β₂-M <3.5 mg/dL and albumin ≥3.5 g/dL
- Stage II: β₂-M <3.5 mg/dL or β₂-M 3.5 - 5.5 mg/dL, and albumin < 3.5 g/dL (neither Stage I or Stage III)
- Stage III: β₂-M >5.5 mg/dL

Multiple myeloma patients are typically classified into one of several myeloma categories. Multiple myeloma can be asymptomatic or symptomatic. Asymptomatic myeloma patients do not show related organ or tissue impairment or symptoms. Myeloma related organ or tissue impairment includes hypercalcemia, impaired kidney function, anemia and bone lesions. Asymptomatic myeloma includes smoldering multiple myeloma (SMM), indolent multiple myeloma (IMM) and Stage I of multiple myeloma. Smoldering multiple myeloma is characterized by monoclonal protein and slightly increased number of plasma cells in the bone marrow. Indolent multiple myeloma is characterized by small amounts of monoclonal protein or increased number of plasma cells in the bone marrow.

Multiple myeloma patients are also characterized by their disease status. Disease status is determined based on whether the patient has already received therapy and if so, the outcome. Patients with newly diagnosed disease are individuals who have myeloma that has yet been treated. Patients who have received therapy fall into several categories:
• *Responsive disease*: refers to myeloma that is responding to therapy. There has been a decrease in M protein of at least 50%.

- *Stable disease:* refers to myeloma that has not responded to treatment (i.e., the decrease in M protein has not reached 50%), but has not progressed (gotten worse).
- *Progressive disease*: refers to active myeloma that is worsening (i.e., increasing M protein and worsening organ or tissue impairment). In most cases, relapsed and/or refractory disease can be considered to be progressive disease.
- *Relapsed disease:* refers to myeloma disease that initially responded to therapy but has then begun to progress again. Patients may be further classified as having relapsed after initial therapy or after subsequent therapy.
- *Refractory disease:* refers to myeloma that has not responded to initial therapy, as well as relapsed myeloma that does not respond to subsequent treatment. In this last instance, the myeloma may also be referred to as relapsed and refractory disease.

The present invention provides methods for treating a subject with multiple myeloma comprising administering to the subject an effective amount of a compound according to Formula I, or its pharmaceutically acceptable salt. The multiple myeloma being treated can be in any of the stages, categories and disease status described above.

In accordance with the methods of the present invention, a compound according to Formula I, or its pharmaceutically acceptable salt, can be used alone or in combination with at least one other therapeutic agent used to reduce one or more symptoms of multiple myeloma. The compound according to Formula I, or its pharmaceutically acceptable salt, can be administered concurrently with the other therapeutic agent, which can be part of the same composition or in a different composition from that comprising the compound according to Formula I, or its pharmaceutically acceptable salt. Alternatively, the compound according to Formula I, or its pharmaceutically acceptable salt, can be administered prior to or subsequent to administration of the other therapeutic agent. A compound according to Formula I, or its pharmaceutically acceptable salt, can be administered through the same or different administration route as the other therapeutic agent. Such therapeutic agent can be chemotherapeutic agents, supportive therapeutic agents or a combination thereof.

As used herein, a "chemotherapeutic agent" is an agent that is toxic to cancer cells. Examples of chemotherapeutic agents that can be used in the present invention include bortezomib (Velcade^{®}, Millennium), melphalan, predisone, vincristine, carmustine, cyclophosphamide, dexamathasone, thalidomide, doxorubicin, cisplatin, etoposide and cytarabine. In a specific embodiment, a compound according to Formula I, or its pharmaceutically acceptable salt is used in combination with bortezomib (Velcade^{®}). In another specific embodiment, a compound according to Formula I, or its pharmaceutically acceptable salt, is used in combination with melphalan.

A "supportive therapeutic agent" is an agent mainly for reducing the symptoms and complications of multiple myeloma. Examples of supportive therapeutic agent include but not limited to bisphosphonates, growth factors, antibiotics, diuretics, and analgesics.

Examples of antibiotics include sulfa drugs, pencillins (e.g., Benzyl penicillin, P-hydroxybenzyl penicillin, 2-pentenyl penicillin, *N*-heptyl penicillin, phenoxymethyl penicillin, Phenethicillin, Methicillin, Oxacillin, Cloxacillin, Dicloxacillin, Flucloxacillino, Nafcillin, Ampicillin, Amoxicillin, Cyclacillin, Carbenicillin, Ticarcillin, Piperacillin, Azlocillin, Meczlocillin, Mecillinam, Amdinocillin), Cephalosporin and derivatives thereof (e.g, Cephalothin, Cephapirin, Cephacetrile, Cephazolin, Caphalexin, Cephandine, Cefadroxil, Cefamandol, Cefuroxime, Ceforanide, Cefoxitin, Cefotetan, Cefaclor, Cefotaxime, Ceftizoxime, Ceftrioxone, Ceftazidime, Moxalactam, Cefoperazone, Cefixime, Ceftibuten and Cefprozil), Oxolinic Acid, Amifloxacin, Temafloxacin, Nalidixic Acid, Piromidic Acid, Ciprofloxacin, Cinoxacin, Norfloxacin, Perfloxacin, Rosaxacin, Ofloxacin, Enoxacin, Pipemidic Acid, Sulbactam, Clavulinic Acid, ß-Bromopenicillanic Acid, ß-Chloropenicillanic Acid, 6-Acetylmethylene-Penicillanic Acid, Cephoxazole, Sultampicillin, Formaldehyde Hudrate Ester of Adinocillin and Sulbactam, Tazobactam, Aztreonam, Sulfazethin, Isosulfazethin, Norcardicins, *m-*Carboxyphenyl Phenylacetamidomethylphosphonate, Chlortetracycline, Oxytetracyline, Tetracycline, Demeclocycline, Doxycycline, Methacycline and Minocycline.

Examples of bisphosphonates include etidronate (Didronel), pamidronate (Aredia), alendronate (Fosamax); risedronate (Actonel), zoledronate (Zometa), ibandronate (Boniva).

Examples of diuretics include thiazide derivatives such as amiloride, chlorothiazide, hydrochlorothiazide, methylchlorothiazide, and chlorothalidon.

Examples of growth factors include, granulocyte colony-stimulating factor (G-CSF), granulocyte-macrophage colony-stimulating factor (GM-CSF), macrophage colony-stimulating factor (M-CSF), multi-colony-stimulating factor, erythropoietin, thrombpoietin, Oncostatin M and interleukins.

Examples of analgesics include an opioid (e.g. morphine), a COX-2 inhibitor (e.g., Rofecoxib, Valdecoxib and Celecoxib), salicylates (e.g., ASPIRIN, choline magnesium trisalicylate, salsalate, difunisal and sodium salicylate), propionic acid derivatives (e.g., fenoprofen calcium, ibuprofen, ketoprofen, naproxen and naproxen sodium), indoleacetic acid derivatives (e.g., indomethacin, sulfindac, etodalac and tolmetin), fenamates (e.g., mefenamic acid and meclofenamate), benzothiazine derivatives or oxicams (e.g., mobic or piroxicam) or pyrrolacetic acid (e.g., ketorolac).

As used herein "treating" includes achieving, partially or substantially, one or more of the following results: partially or totally reducing the extent of the disease; ameliorating or improving a clinical symptom or indicator associated with the disease; delaying, inhibiting or preventing the progression of the disease; or partially or totally delaying, inhibiting or preventing the onset or development of disease.

A "subject" is a mammal, preferably a human, but can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

The method of treatment of a subject suffering from multiple myeloma comprises administration to the subject of an effective amount of a compound which is an anilinopyrimidine derivative of Formula (I)

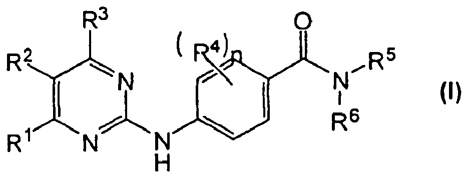

Said compounds are disclosed in WO 02/46171 (Signal Pharmaceuticals Inc.), which are described in particular for the treatment of autoimmune disorders, inflammatory diseases, cardiovascular diseases, infectious diseases, stroke or cancer.

In said compounds according to Formula (I), which include its pharmaceutically acceptable salts thereof, the substituents are defined as follows:
R¹ is either an aryl or heteroaryl optionally substituted with one to four substituents independently selected from R⁷;
R² is hydrogen;
R³ is either hydrogen or lower alkyl;
R⁴ is, in each instance, independently selected from the group consisting of halogen, hydroxy, lower alkyl and lower alkoxy; and wherein n is an integer from 0-4;
R⁵ and R⁶ are the same or different and are independently selected from the group consisting of -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐSO_{c}R⁹ or -(CH₂)ₐSO₂NR⁹R¹⁰; and R⁵ and R⁶ taken together with the nitrogen atom to which they are attached to form a heterocycle or substituted heterocycle;
R⁷ is at each occurrence independently selected from the group consisting of halogen, hydroxy, cyano, nitro, carboxy, alkyl, alkoxy, haloalkyl, acyloxy, sulfanylalkyl, sulfinylalkyl, sulfonylalkyl, hydroxyalkyl, aryl, substituted aryl, alkylaryl, substituted alkylaryl, heterocycloalkyl, substituted heterocycloalkyl, alkylheterocycloalkyl, substituted alkylheterocycloalkyl, -C(=O)OR⁸, -OC(=O)R⁸, -C(=0)NR⁸R⁹, -C(=O)NR⁸OR⁹, -SO_{C}R⁸, -SO_{C}NR⁸R⁹, -NR⁸SO_{C}R⁹, -NR⁸R⁹, -NR⁸C(=O)R⁹, -NR⁸C(=O)(CH₂)_{b}OR⁹, -NR⁸C(=O)(CH₂)_{b}R⁹, -O(CH₂)_{b}NR⁸R⁹ and substituted or unsubstituted heterocycloalkyl fused to substituted or unsubstituted phenyl;
R⁸, R⁹, R¹⁰ and R¹¹ are the same or different and are at each occurrence independently selected from the group consisting of hydrogen, alkyl, substituted alkyl, aryl, substituted aryl, alkylaryl, substituted alkylaryl, heterocycloalkyl, substituted heterocycloalkyl, alkylheterocycloalkyl and substituted alkylheterocycloalkyl;
or R⁸ and R⁹ taken together with the atom or atoms to which they are attached to form a heterocycle or substituted heterocycle;
a and b are the same or different and are at each occurrence independently selected from the group consisting of 0, 1, 2, 3 or 4; and
c is at each occurrence 0, 1 or 2.

In one embodiment of the invention, R¹ is either a substituted or unsubstituted aryl or heteroaryl. When R¹ is substituted, it is substituted with one or more substituents defined below. Preferably, when substituted, R¹ is substituted with a halogen, sulfonyl or sulfonamide.

In another embodiment of the invention, R¹ is selected from the group consisting of a substituted or unsubstituted aryl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl and quinazolinyl.

In another embodiment of the invention, R¹ is a substituted or unsubstituted aryl, preferably a substituted or unsubstituted phenyl. When R¹ is a substituted aryl, the aryl is substituted with one or more substituents defined below.

Preferably, when R¹ is a substituted aryl, it is substituted with a halogen, sulfonyl or sulfonamide.

In another embodiment of the invention, R⁵ and R⁶, taken together with the nitrogen atom to which they are attached form a substituted or unsubstituted nitrogen-containing non-aromatic heterocycle, preferably substituted or unsubstituted morpholinyl, substituted or unsubstituted thiomorpholinyl, substituted or unsubstituted pyrrolidinonyl, substituted or unsubstituted pyrrolidinyl, substituted or unsubstituted piperidinyl, substituted or unsubstituted homopiperidinyl, substituted or unsubstituted piperazinyl, substituted or unsubstituted homopiperazinyl, substituted or unsubstituted hydantoinyl, substituted or unsubstituted tetrahydropyrindinyl, substituted or unsubstituted tetrahydropyrimidinyl, substituted or unsubstituted oxazolidinyl, substituted or unsubstituted thiazolidinyl, substituted or unsubstituted indolinyl, substituted or unsubstituted isoindolinyl, substituted or unsubstituted tetrahydroquinolinyl or substituted or unsubstituted tetrahydroisoquinolinyl.

When R⁵ and R⁶, taken together with the nitrogen atom to which they are attached form a substituted or unsubstituted piperazinyl, a substituted or unsubstituted piperadinyl or a substituted or unsubstituted morpholinyl, the substituted piperazinyl, substituted piperadinyl or substituted morpholinyl is substituted with one or more substituents defined below.

Preferably, when substituted, the substituent is alkyl, amino, alkylamino, alkylether, acyl, pyrrolidinyl or piperidinyl.

In one embodiment of the invention, R², R³ and R⁴ are hydrogen, and the compounds of this invention has the following Formula (II):

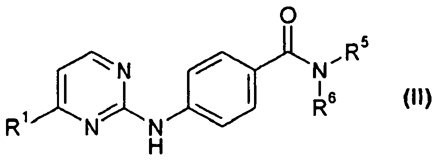

In a more specific embodiment of the invention, R¹ is a phenyl optionally substituted with R⁷, and having the following Formula (III):

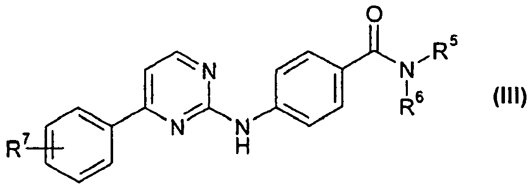

In still a further embodiment of the invention, R⁷ is at the para position of the phenyl ring , as represented by the following Formula (IV):

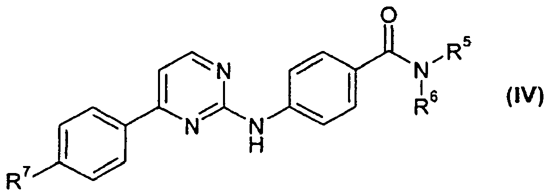

In still a further embodiment of the invention, in the anilinopyrimidine derivatives is compound A : 1-(4-{4-[4-(4-Chloro-phenyl)-pyrimidin-2-ylamino]-benzoyl}-piperazin-1-yl)-ethanone.

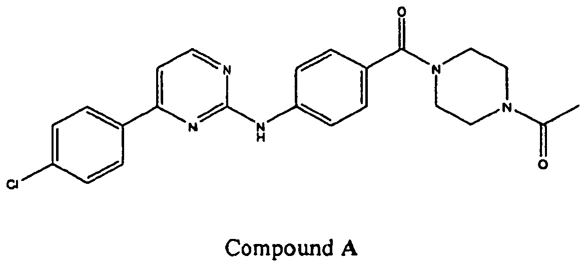

In another embodiment, the invention is a compound represented by Structural Formula (I), (II), (III) or (IV) or a pharmaceutically acceptable salt thereof, provided that compound **A** or a pharmaceutically acceptable salt thereof is excluded.

Compounds according to Formulae I-IV and compound **A** can be prepared by methods described in WO 02/46171 A2, (Signal Pharmaceuticals Inc.) the entire contents of which are incorporated herein by reference.

In one embodiment, the subject being treated with a compound according to Formula I, or its pharmaceutically acceptable salt, alone or in combination with other therapeutic agents, is undergoing radiation therapy. In another embodiment, the subject being treat with a compound according to Formula I, or its pharmaceutically acceptable salt, alone or in combination with other therapeutic agents, is in preparation for a stem cell transplantation. A compound according to Formula I, or its pharmaceutically acceptable salt, alone or in combination with other therapeutic agents can be used as an induction therapy to reduce the tumor burden prior to a stem cell transplantation. In another embodiment, the subject being treat with a compound according to Formula I, or its pharmaceutically acceptable salt, alone or in combination with other therapeutic agents, is undergoing a stem cell transplantation.

When a compound according to Formula I, or its pharmaceutically acceptable salt, is named or depicted by structure, it is to be understood that solvates or hydrates of the compound are also included. "Solvates" refer to crystalline forms wherein solvent molecules are incorporated into the crystal lattice during crystallization. Solvate may include water or nonaqueous solvents such as ethanol, isopropanol, DMSO, acetic acid, ehtanolamine, and EtOAc. Solvates, wherein water is the solvent molecule incorporated into the crystal lattice, are typically referred to as "hydrates". Hydrates include stoichiometric hydrates as well as compositions containing variable amounts of water.

Furthermore, the invention provides pharmaceutical compositions comprising a compound according to Formula I, or a pharmaceutically acceptable salt thereof, as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, a compound according to Formula I can be combined as the active ingredient in admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. An active compound according to Formula I can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

A compound according to Formula I may also be administered parenterally. Solutions or suspensions of the active compound can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably a compound according to Formula I is administered orally.

The term "effective amount" is the quantity of compound in which a beneficial clinical outcome is achieved when the compound is administered to a subject. A "beneficial clinical outcome" includes amelioration or improvement of the clinical symptoms of the disease; prevention, inhibition or a delay in the recurrence of symptom of the disease or of the disease itself and/or an increase in the longevity of the subject compared with the absence of the treatment, or prevention, inhibition or a delay in the progression of symptom of the disease or of the disease itself. The precise amount of compound (or other therapeutic agent) administered to a subject will depend on the type and severity of the disease or condition and on the characteristics of the subject, such as general health, age, sex, body weight and tolerance to drugs. It will also depend on the degree, severity and type of disease. The skilled artisan will be able to determine appropriate dosages depending on these and other factors. When co-administered with another therapeutic agent, an "effective amount" of the second agent will depend on the type of drug used. The effective dosage may vary depending on the mode of administration.

A compound according to Formula I can be administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

The invention is illustrated by the following examples which are not intended to be limiting in any way.

### EXPERIMENTAL

### 1. Cell lines and cell proliferation assay.

### Characterization and maintenance

Myeloma cell lines were passaged every 3-4 days in RPMI640 and 10% fetal calf serum (FCS). For the IL-6 dependent cell lines, 2 ng/ml of freshly thawed IL-6 (Abcys, France) was added at the beginning of the culture. Each cell line has unique phenotypic and HLA characteristics that make it possible to periodically check their identity.

The following cell lines were obtained in B. Klein's laboratory: XG-1, XG-2, XG-3, XG-4, XG-6, XG-7, XG-11, XG-12, XG-13, XG-14, XG-19, XG-20. (Zhang, *et al.,* 1994; Rebouissou, *et al.,* 1998)

RPMI8226 and L363 cell lines were purchased from ATCC.

### Cell Proliferation Assay

Myeloma cells were harvested in the exponential growth phase (at day 3 after culture passage when the cell concentration is around 5 x 10⁵ cells/ml). Cells were washed twice, cultured for 3 hours in RPMI1640 and 10% FCS, washed twice. This makes it possible to remove cell-bound growth factors (in particular recombinant IL-6). Then cells are plated in 96-well flat-bottomed microplates at various concentrations depending on their doubling time:
- 5 x 10³ cells/well in 100 µl for the cell lines: XG-1, XG-7
- 10⁴ cells/well in 1000 µl for the cell lines: XG-2, XG-4, XG-6, XG-11, PRMI8226, L363
- 2 x 10⁴ cells/well in 100 µl for the cell lines: XG-3, XG-12, XG-13, XG-14, XG-19, XG-20

The culture medium used were:
- PRMI1640, 10% FCS and 0.5 ng/ml of IL-6 for XG-1, XG-2, XG-3, XG-4, XG-6, XG-7, XG-11, XG-12, XG-13, XG-19, XG-20 cells
- X-VIVO20 and 0.5 ng/ml IL-6 for XG-14 cells
- PRMI1640 and 10% FCS for RPMI8226 and L363 cells

The diluted compounds, DMSO or control culture medium were added in 100 µl at the start of the culture. Three culture wells were done for one compound dilution and the experiments were done twice (finally six culture wells for one compound dilution).

Cells were cultured for 4 days. Eight hours before the end of the culture, 0.5 µCi of tritiated thymidine were added in 50 µl of RPMI1640 and 10% FCS. Cells were then extracted with a cell collector and the thymidine incorporation was determined using a beta cell counter.

A putative toxicity of DMSO was evaluated by adding a DMSO group containing the highest DMSO concentration (1:333 DMSO corresponding to the compound).

Stock solutions of Velcade (3mM) and Dexamethasone (10 mM) were prepared in DMSO. These compounds were diluted in RPMI1640 and 10% FCS - VELCADE (1 µM final concentration) and DEX (10⁻⁶ M) final concentration) - and were used as positive control for myeloma cell proliferation inhibition.

### 2. Detection of apoptosis of myeloma cell lines.

Apoptotic cells were detected using fluorescein isothiocyanate-labelled annexin V (FITC-annexin-V, Boehringer Mannheim). Annexin V has a high affinity for phosphatidylserine present on the outer cytoplasmic membrane of apoptotic cells. Cells were washed, labeled with FITC-annexin-V according to the manufacturer's recommendations and analyzed with a FACScan flow cytometer using Cell Quest software (Becton Dickinson, Moutain View, CA, USA).

### 3. Measurements of the cell cycle of myeloma cell lines.

The cell cycle distribution of the cell lines was assessed by flow cytometry analysis by propidium iodide (PI) and bromodeoxyuridine (BrdU) double-staining. Thirty minutes before stopping the culture, BrdU (10 µM) were added to the cultures and then the cells were collected by centrifugation, washed twice in phosphate buffer saline (PBS) and fixed in 70% ethanol for 20 minutes at room temperature. After two washes with PBS, cells were resuspended in 50 µL of 3N HCl, 0.5% Tween 20 and incubated 20 minutes at 20 °C to denature DNA. The cells were then recovered by centrifugation, resuspended in 250 µL of 10 mM sodium tetraborate to neutralize the reaction, washed twice with PBS, 0.05% Tween 20, and incubated with 20 µL of anti-BrdU-FITC (BD Biosciences). After two additional washes, the cells were resuspended in 500 µL of PBS, 0.05% Tween 20 containing 10 µg/ml PI. The fluorescence of FL1-H (BrdU) and FL2-A (PI) were analyzed on a FACScan flow cytometer using Cell Quest software (Becton Dickinson).

### 4. Statistical analysis.

For each experiment, the mean tritiated thymidine incorporations (mean values determined on three culture wells) were plotted against the logarithmic concentrations of the compound. The curves were fitted with exponential regression, which was determined using the concentrations defining the inhibition response (usually 3-4 concentrations).

The concentrations yielding 50% and 90% inhibition of the proliferation of myeloma cells were determined with these regression curves.

### 5. Bone marrow cells

Bone marrow cells from 10 patients with multiple myeloma (5 patients at diagnosis and 5 relapsing patients) were harvested and mononuclear cells isolated by ficoll hypaque centrifugation.

Bone marrow mononuclear cells were cultured at 5 x 10⁵ cells/ml in RPMI1640 and 5% of fetal calf serum with culture medium as control, with highest concentration of DMSO and three concentrations of compound **A**. The three concentration (1 µM, 3.3 µM and 10 µM) used for compound A were the mean concentration determined on 10 myeloma cell lines yielding 10%, 50% or 90% inhibition.

At day 5, cell numbers were counted and the percentage and counts of myeloma cells were determined by FACS staining using an anti-CD138 monoclonal antibody. This made it possible to assay for viable myeloma cells as CD138 is lost on pre-apoptotic myeloma cells (Jourdan, M. et al. (1998) Br. J. Haematol., 100, 637-648). The percentage and count of CD34 hematopoietic progenitors was also determined with an anti-CD34 monoclonal antibody and FACS labeling.

### Example 1 Inhibition of the Proliferation of Myeloma Cells by Compound A

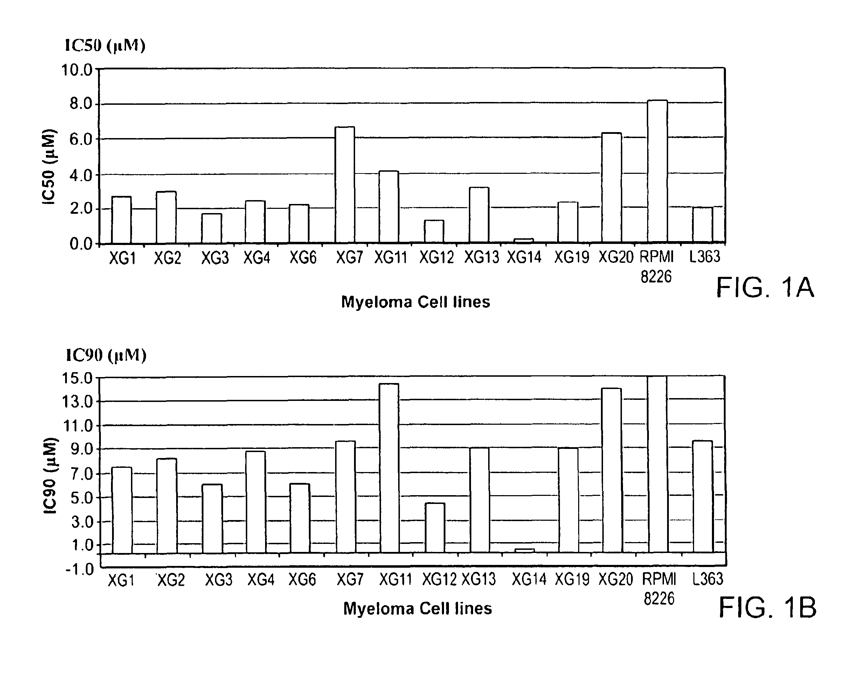

A compound according to Formula I, 1-(4-{4-[4-(4-chlorophenyl)-pyrimidin-2-ylamino]-benzoyl}-piperazin-1-yl)-ethanone (compound A) was diluted in DMSO (Sigma chemicals) in order to obtain a stock solution of 3 mM. The stock solution was aliquoted and stored at -80 °C until use. The compound was thawed, briefly warmed to 37 °C in a water bath and diluted in RPMI160 and 10% FCS to obtain a concentration of 10 µM. A suitable amount of the 10 µM solution was added to the culture wells containing myeloma cells to obtain the following concentrations of compound **A**: 10 µM, 3.33 µM, 1.11 µM, 0.37 µM, 0.12 µM, 0.04 µM. As shown in Figure 1, Compound **A** exhibited a full inhibition of the proliferation of 13 out 14 human myeloma cells.

### Example 2 Induction of Myeloma Cell Apoptosis and Inhibition of Myeloma Cell Cycles by Compound A

Two myeloma cell lines (XG3-3 and XG-12) that are sensitive to compound **A** (1-(4-{4-[4-(4-chlorophenyl)-pyrimidin-2-ylamino]-benzoyl}-piperazin-1-yl)-ethanone) were selected for the study. To assess the effect of compound **A** on myeloma cell survival, the percentage apoptotic cells was determined using binding of annexin V to apoptotic cells. The effect of compound **A** on the cell cycle was assessed by evaluating the percentage of cells in the S phase of the cell cycle by incorporation of BrdU-FITC. Apoptosis, cell cycle and cell numeration were evaluated daily on a four day culture period on the cell lines using the IC₁₀, IC₅₀ and I₉₀ respective doses of compound **A**.

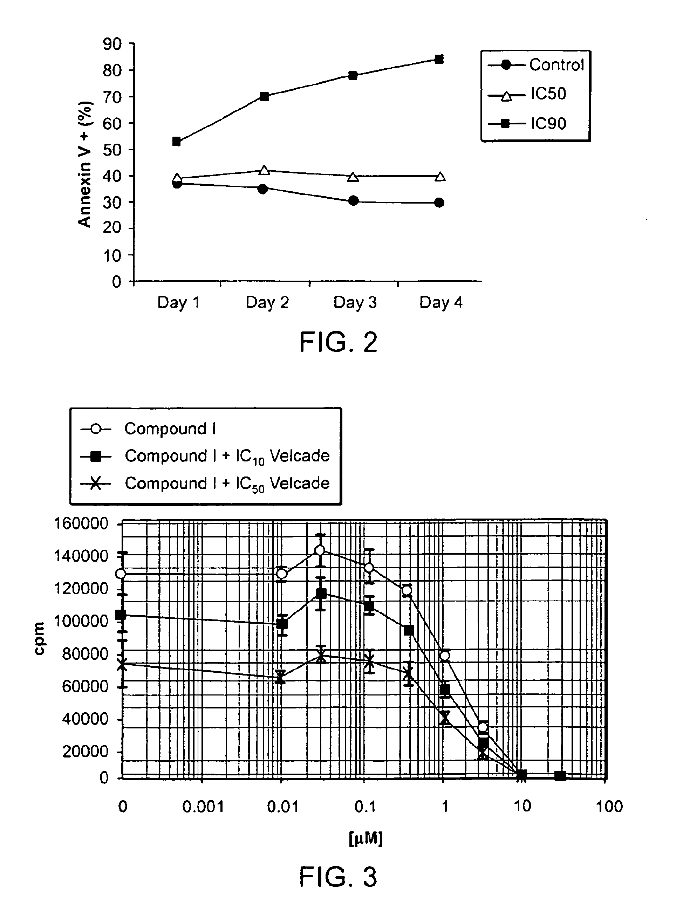

As shown in Table 1 and FIG. 2, Compound **A** induced apoptosis of myeloma cell lines. The apoptosis was dose- and time-dependent. Compound **A** also induced a decrease in the number of cell in the S phase of the cell cycle of the myeloma cell lines, shown in Table 1.

**Table 1. Percentage of apoptotic cells, percentage of cells in the S phase and cell counts for XG-12 or XG-3 myeloma cell lines treated with compound A at IC₁₀, IC₅₀ or IC₉₀ concentrations on day 3 of the cell culture.**

| | | Co | IC₁₀ | IC₅₀ | IC₉₀ |
|---|---|---|---|---|---|
| **XG-12** | Annexin V (%) | 24.9 | ND | 39.5 | 79.8 |
| | S phase (%) | 40.8 | ND | 37.3 | 6.8 |
| | Cells (x 10⁶/ml) | 0.41 | ND | 0.35 | 0.11 |
| **XG-3** | Annexin V (%) | 25.5 | 29.2 | 30.7 | 48.2 |
| | S phase (%) | 35.3 | 32.1 | 29.2 | 15.3 |
| | Cells (x 10⁶/ml) | 0.83 | 0.68 | 0.47 | 0.29 |

### Example 3 Inhibition of the Proliferation of Myeloma Cells by Compound A and Velcade or Melphalan

Cell lines that have been shown to be the most sensitive (XG12 and XG3) or the less sensitive (XG7 and L363) to compound **A** (1-(4-{4-[4-(4-chlorophenyl)-pyrimidin-2-ylamino]-benzoyl}-piperazin-1-yl)-ethanone) were used for the study. The concentration yielding 10% (IC₁₀) or 50% (IC₅₀) inhibition of the myeloma cell lines were determined and the data are listed in Table 2.

**Table 2. Inhibitory effects of melphalan and Velcade in myeloma cell lines.**

| | | XG12 | XG3 | XG7 | L363 |
|---|---|---|---|---|---|
| Melphalan | IC₁₀ (µM) | 0.318 | 0.490 | 0.409 | 0.110 |
| | IC₅₀ (µM) | 1.234 | 1.680 | 1.181 | 0.457 |
| Velcade | IC₁₀ (µM) | 0.0016 | 0.0016 | 0.0014 | 0.0049 |
| | IC₅₀ (µM) | 0.0039 | 0.0022 | 0.0035 | 0.0084 |

Compound **A** was tested in combination with melphalan or Velcade. Increasing concentration of compound **A** (0, 0.01, 0.03, 0.12, 0.37, 1.1, 3.1, 10, 30 µM) were applied on 4 myeloma cell lines alone or in combination with Velcade or melphalan at concentration yielding around 10% or 50% inhibition.

The concentration of compound **A** yielding 50% or 90% inhibition of the proliferation of the myeloma cell lines in the absence or in the presence of Velcade was determined, shown in Table 3 and FIG. 3. An additive effect was observed when compound **A** was used in combination with Velcade as concentrations of compound **A** inducing 50% or 90% inhibition of myeloma cell proliferation were similar with or without Velcade.

**Table 3. Inhibitory effects of compound A with or without Velcade.**

| | | Compound **A** | Compound **A** + Velcade (IC₁₀) | Compound **A** + Velcade (IC₅₀) |
|---|---|---|---|---|
| XG3 | IC₅₀ | 1.485 | 1.313 | 1.368 |
| | IC₉₀ | 6.353 | 5.944 | 5.869 |
| XG12 | IC₅₀ | 0.974 | 1.256 | 0.611 |
| | IC₉₀ | 3.918 | 4.278 | 2.915 |
| XG7 | IC₅₀ | 6.363 | 5.921 | 5.611 |
| | IC₉₀ | 9.330 | 9.842 | 9.016 |
| L363 | IC₅₀ | 2.329 | 2.744 | 3.910 |
| | IC₉₀ | 10.102 | 9.133 | 10.474 |

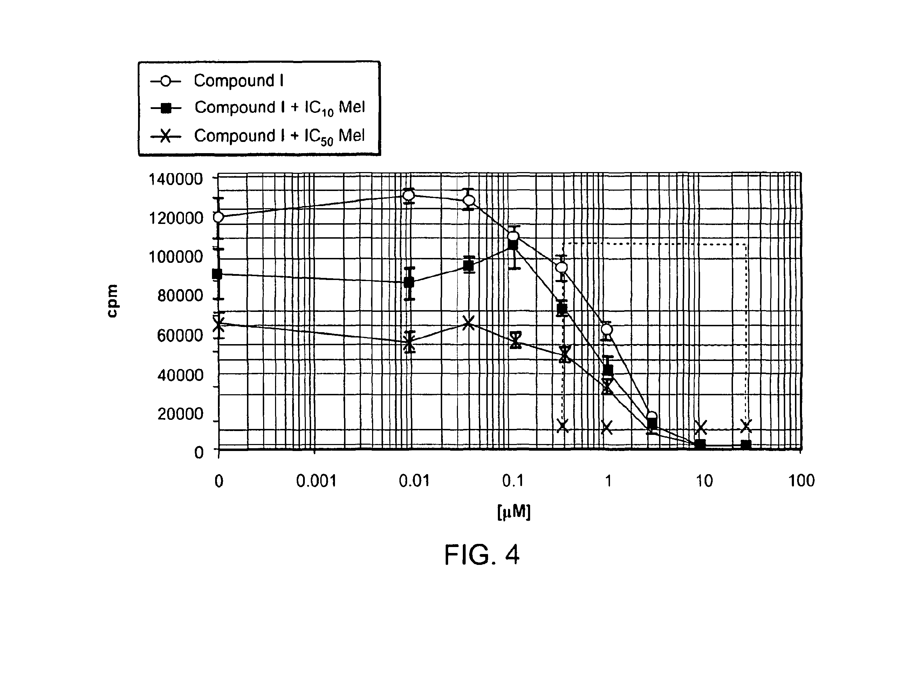

Similar combination study was performed in the presence of increasing concentration of compound **A** and fixed concentration of Melphalan (IC₁₀ or IC₅₀). Data are shown in Table 4 and FIG. 4. An additive effect was observed when compound **A** was used in combination with Melphalan as concentrations of compound **A** inducing 50% or 90% inhibition of myeloma cell proliferation were similar with or without Melphalan.

**Table 4. Inhibitory effects of compound A with or without Melphalan.**

| | | Compound **A** | Compound **A** + Melphalan (IC₁₀) | Compound **A** + Melphalan (IC₅₀) |
|---|---|---|---|---|
| XG3 | IC₂₀ | 1.485 | 1.492 | 1.690 |
| | IC₉₀ | 6.353 | 6.243 | 6.098 |
| XG12 | IC₅₀ | 0.974 | 0.932 | 1.072 |
| | IC₉₀ | 3.918 | 3.539 | 3.795 |
| XG7 | IC₅₀ | 6.363 | 6.037 | 7.561 |
| | IC₉₀ | 9.330 | 9.202 | 9.630 |
| L363 | IC₅₀ | 1.791 | 1.962 | 1.488 |
| | IC₉₀ | 9.412 | 9.430 | 8.547 |

### Example 4 Effect of Compound A on the survival of primary myeloma cells, non myeloma cells and CD34⁺ hematopoietic stem cells.

Bone marrow mononuclear cells from 5 patients with newly diagnosed multiple myeloma or from 5 patients with relapsing multiple myeloma were cultured for 5 days with culture medium and 10 % fetal calf serum. Cells were treated with 10⁻⁶ M dexamethasone, 1 µM Velcade or compound A (1-(4-{4-[4-(4-chlorophenyl)-pyrimidin-2-ylamino]-benzoyl}-piperazin-1-yl)-ethanone) at mean IC₁₀, IC₅₀ or IC₉₀ concentrations. At the end of culture, cells were counted. Viable myeloma cells were determined using FACS labeling with anti-CD138 monoclonal antibody, as well as viable hematopoietic stem cells using an anti-CD34 monoclonal antibody. Results are expressed as the percentage of viable myeloma cells, non-myeloma cells, or CD34 cells compared to the control culture group without inhibitor.

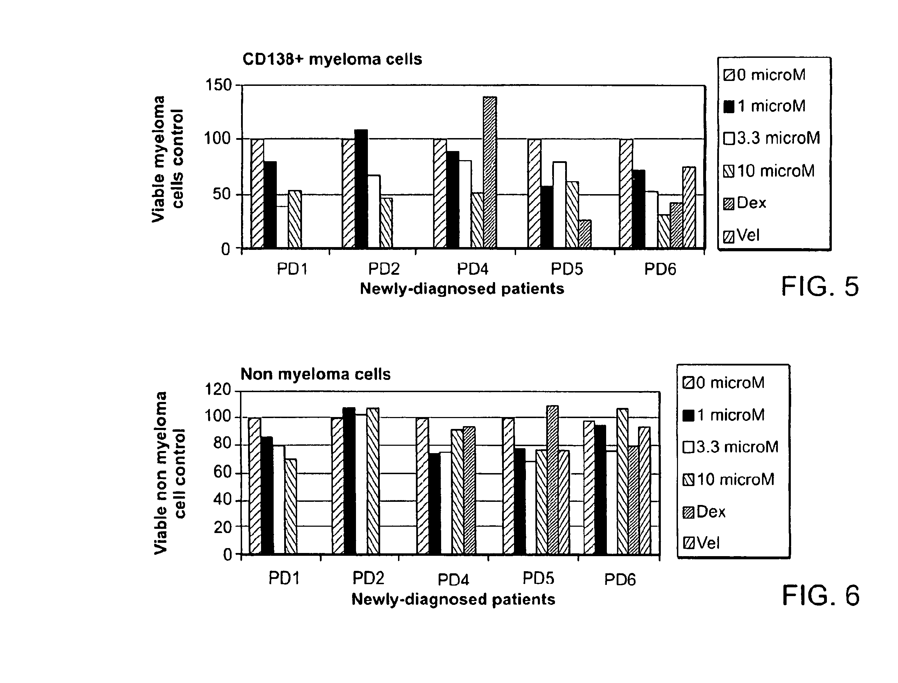

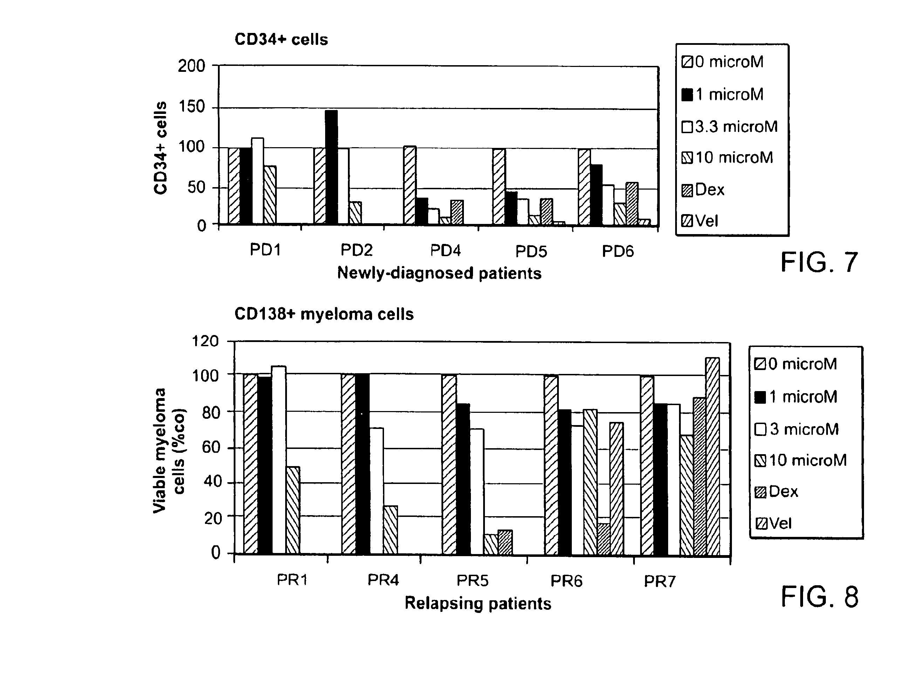

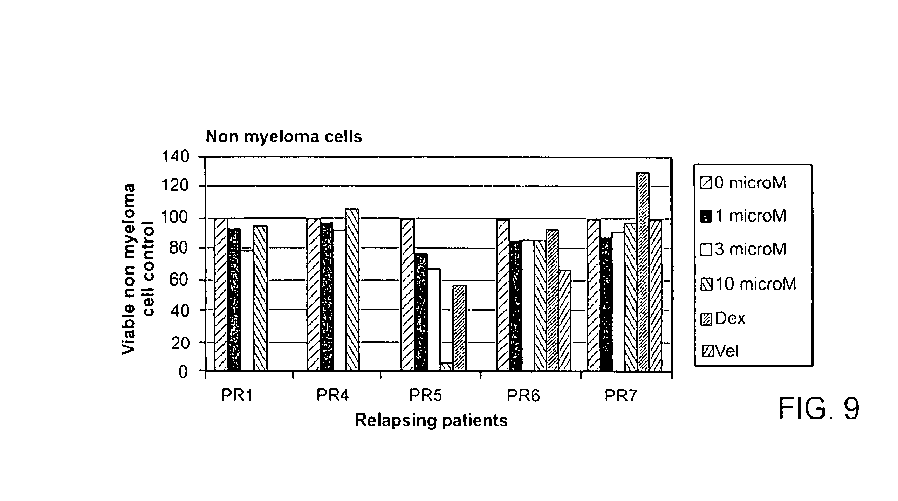

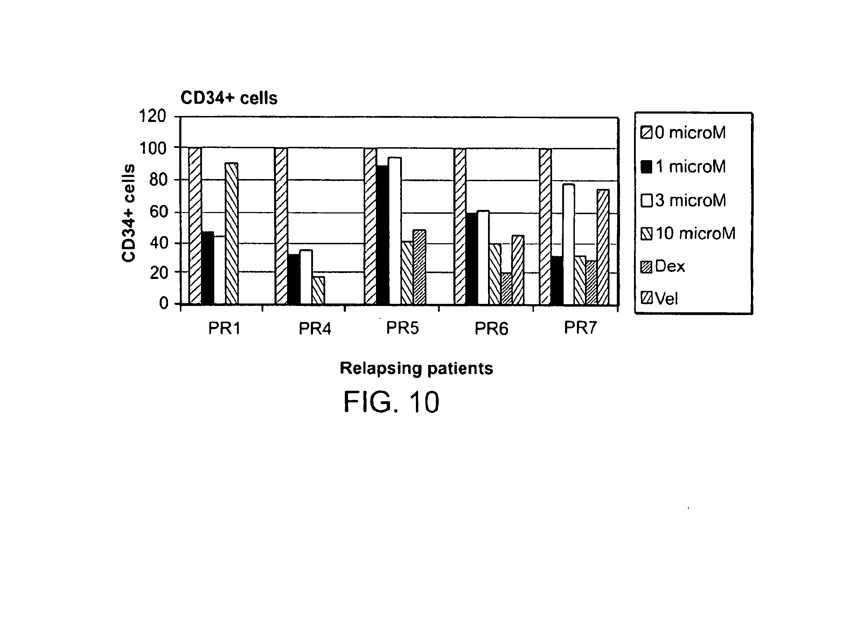

As shown in FIGs 5-10, compound A inhibits the survival of primary myeloma cells without affecting the survival of other bone marrow cells except that of hematopoietic precursors for some patients.

While this invention has been particularly shown and described with references to example embodiments thereof, it will be understood by those skilled in the art that various changes in form and details may be made therein without departing from the scope of the invention encompassed by the appended claims.

## Claims

1. A compound according to Formula I: 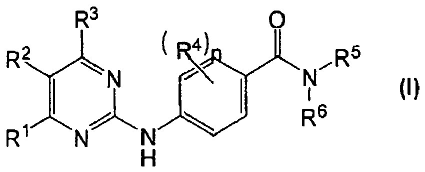 or a pharmaceutically acceptable salt thereof, wherein
R¹ is either aryl or heteroaryl optionally substituted with one to four substituents independently selected from R⁷;
R² is hydrogen;
R³ is either hydrogen or lower alkyl;
R⁴ is, in each instance, independently selected from the group consisting of halogen, hydroxy, lower alkyl and lower alkoxy; wherein n is an integer from 0 to 4;
R⁵ and R⁶ are the same or different and are independently selected from the group consisting of -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐNR⁹C(=O)R¹⁰, -(CH₂)ₐSO_{c}R⁹ and -(CH₂)ₐSO₂NR⁹R¹⁰;
or R⁵ and R⁶ taken together with the nitrogen atom to which they are attached to form an optionally substituted heterocycle;
R⁷ is at each occurrence independently selected from the group consisting of halogen, hydroxy, cyano, nitro, carboxy, alkyl, alkoxy, haloalkyl, acyloxy, sulfanylalkyl, sulfinylalkyl, sulfonylalkyl, hydroxyalkyl, aryl, substituted aryl, alkylaryl, substituted alkylaryl, heterocycloalkyl, substituted heterocycloalkyl,
alkylheterocycloalkyl, substituted alkylheterocycloalkyl, -C(=O)OR⁸ -OC(=O)R⁸, -C(=O)NR⁸R⁹, -C(=O)NR⁸OR⁹, -SO_{C}R⁸, -SO_{C}NR⁸R⁹, -NR⁸SO_{C}R⁹ -NR⁸R⁹, -NR⁸C(=O)R⁹, -NR⁸C(=O)(CH₂)_{b}OR⁹, -NR⁸C(=O)(CH₂)_{b}R⁹, -O(CH₂)_{b}NR⁸R⁹ and heterocycloalkyl fused to phenyl;
R⁸, R⁹, R¹⁰ and R¹¹ are the same or different and are at each occurrence independently selected from the group consisting of hydrogen, alkyl, substituted alkyl, aryl, substituted aryl, alkylaryl, substituted alkylaryl, heterocycloalkyl, substituted heterocycloalkyl, alkylheterocycloalkyl and substituted alkylheterocycloalkyl;
or R⁸ and R⁹ taken together with the atom or atoms to which they are attached form an optionally substituted heterocycle;
a and b are the same or different and are at each occurrence independently selected from the group consisting of 0, 1, 2, 3 and 4; and
c is at each occurrence 0, 1 or 2,
for use in the treatment of multiple myeloma.

2. A compound for use according to claim 1 selected from the formulae (II), (III) and (IV): 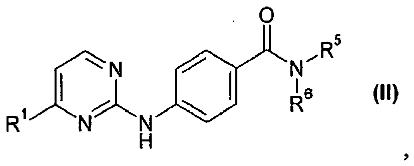 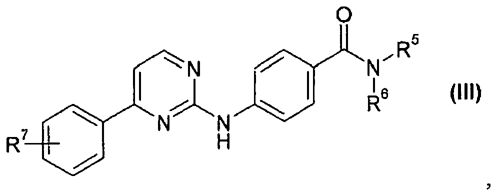 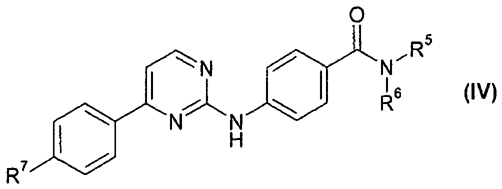 or a pharmaceutically acceptable salt thereof, wherein R¹, R⁵, R⁶ and R⁷ arc as defined in claim 1;

3. A compound for use according to claims 1 or 2, wherein R⁵ and R⁶, taken together with the nitrogen atom to which they are attached form an optionally substituted nitrogen-containing non-aromatic heterocycle.

4. A compound for use according to claim 3, wherein the nitrogen-containing non-aromatic heterocycle is selected from the group consisting of morpholinyl, thiomorpholinyl, pyrrolidinonyl, pyrrolidinyl, piperidinyl, homopiperidinyl, piperazinyl, homopiperazinyl, hydantoinyl, tetrahydropyridinyl, tetrahydropyrimidinyl, oxazolidinyl, thiazolidinyl, indolinyl, isoindolinyl, tetrahydroquinolinyl and tetrahydroisoquinolinyl.

5. A compound for use according to any one of claims 1 to 4, wherein R¹ is either aryl or heteroaryl.

6. A compound for use according to any one of claims 1 to 5, wherein R¹ is selected from the group consisting of aryl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl and quinazolinyl.

7. A compound for use according to any one of claims 1 to 6, wherein R¹ is phenyl.

8. A compound for use according to any one of claims 3 or 4, wherein the nitrogen-containing heterocycle is piperazinyl, piperidinyl, or morpholinyl.

9. Compound A for use according to claim 1, named :1-(4-{4-(4-Chloro-phenyl)-pyrimidin-2-ylamino]-benzoyl}-piperazin-1-yl)-ethanone, and represented by the structural formula: 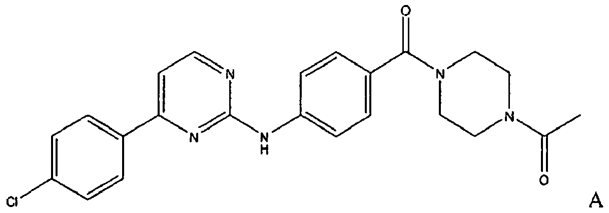 or a pharmaceutically acceptable salt thereof.

10. A compound for use according to claims 1 to 9, wherein the multiple myeloma is stage I multiple myeloma, stage II multiple myeloma, stage III multiple myeloma, asymptomatic multiple myeloma, symptomatic myeloma, newly diagnosed multiple myeloma, responsive multiple myeloma, stable multiple myeloma, progressive multiple myeloma, relapsed multiple myeloma, or refractory multiple myeloma.

11. A compound for use according to claim 10, wherein the asymptomatic multiple myeloma is smoldering multiple myeloma or indolent multiple myeloma.

12. A pharmaceutical composition comprising a compound of Formula (I), (II), (III) or A according to claims 1 to 9 or a physiologically acceptable salt thereof, and further comprising an effective amount of at least one therapeutic agent selected from the group consisting of bortezomib (velcade), melphalan, prednisone, vincristine, carmustine, cyclophosphamide, dexamathasone, thalidomide, doxorubicin, cisplatin, etoposide and cytarabine.

13. A pharmaceutical composition according to claim 12, wherein the therapeutic agent is selected from velcade or melphalan.

14. A compound for use according to claims 1 to 9, for treating multiple myeloma in a subject undergoing radiation therapy, in preparation for a stem cell transplantation, or undergoing a stem cell transplantation.

## Patentansprüche

1. Verbindung gemäß Formel I: 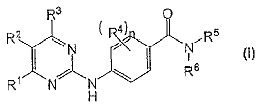 oder ein pharmazeutisch akzeptables Salz davon, wobei R¹ entweder Aryl oder Heteroaryl ist, gegebenenfalls substituiert mit einem bis vier Substituenten unabhängig voneinander ausgewählt aus R⁷;
R² Wasserstoff is;
R³ entweder Wasserstoff oder Niederalkyl ist;
R⁴ in jedem Fall unabhängig voneinander ausgewählt ist aus der Gruppe bestehend aus Halogen, Hydroxy, Niederalkyl und Niederalkoxy; wobei n eine ganze Zahl von 0 bis 4 ist;
R⁵ und R⁶ gleich oder unterschiedlich sind und unabhängig voneinander ausgewählt sind aus der Gruppe bestehend aus -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐNR⁹C(=O)R¹⁰, -(CH₂)ₐSO_{c}R⁹ und -(CH₂)ₐSO₂NR⁹R¹⁰;
oder R⁵ und R⁶ zusammen mit dem Stickstoffatom, an das sie gebunden sind, einen gegebenenfalls substituierten Heterocyclus bilden;
R⁷ bei jedem Auftreten unabhängig voneinander ausgewählt ist aus der Gruppe bestehend aus Halogen, Hydroxy, Cyano, Nitro, Carboxy, Alkyl, Alkoxy, Halogenalkyl, Acyloxy, Sulfanylalkyl, Sulfinylalkyl, Sulfonylalkyl, Hydroxyalkyl, Aryl, substituiertes Aryl, Alkylaryl, substituiertes Alkylaryl, Heterocycloalkyl, substituiertes Heterocycloalkyl, Alkylheterocycloalkyl, substituiertes Alkylheterocycloalkyl, -C(=O)OR⁸, -OC(=O)R⁸, -C(=O)NR⁸R⁹, -C(=O)NR⁸OR⁹, -SO_{c}R⁸, -SO_{c}NR⁸R⁹, -NR⁸SO_{c}R⁹ -NR⁸R⁹, -NR⁸C(O)R⁹, -NR⁸C(=O)(CH₂)_{b}OR⁹, -NR⁸C(=O)(CH₂)_{b}R⁹, -O(CH₂)_{b}NR⁸R⁹ und mit Phenyl anelliertes Heterocycloalkyl;
R⁸, R⁹, R¹⁰ und R¹¹ gleich oder unterschiedlich sind und bei jedem Auftreten unabhängig voneinander ausgewählt sind aus der Gruppe bestehend aus Wasserstoff, Alkyl, substituiertes Alkyl, Aryl, substituiertes Aryl, Alkylaryl, substituiertes Alkylaryl, Heterocycloalkyl, substituiertes Heterocycloalkyl, Alkylheterocycloalkyl und substituiertes Alkylheterocycloalkyl;
oder R⁸ und R⁹ zusammen mit dem Atom oder den Atomen, an das/die sie gebunden sind, einen gegebenenfalls substituierten Heterocyclus bilden;
a und b gleich oder unterschiedlich sind und bei jedem Auftreten unabhängig voneinander ausgewählt sind aus der Gruppe bestehend aus 0, 1, 2, 3 und 4; und
c bei jedem Auftreten 0, 1 oder 2 ist,
zur Verwendung bei der Behandlung von multiplem Myelom.

2. Verbindung zur Verwendung nach Anspruch 1, ausgewählt aus den Formeln (II), (III) und (IV): 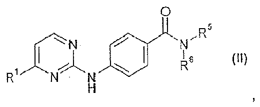 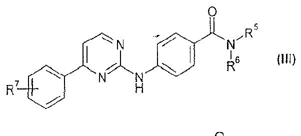 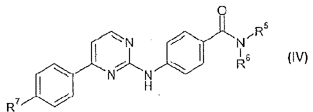 oder ein pharmazeutisch akzeptables Salz davon, wobei R¹, R⁵, R⁶ und R⁷ wie in Anspruch 1 definiert sind.

3. Verbindung zur Verwendung nach Anspruch 1 oder 2, wobei R⁵ und R⁶ zusammen mit dem Stickstoffatom, an das sie gebunden sind, einen gegebenenfalls substituierten Stickstoff-enthaltenden nicht aromatischen Heterocyclus bilden.

4. Verbindung zur Verwendung nach Anspruch 3, wobei der Stickstoff-enthaltende nicht aromatische Heterocyclus ausgewählt ist aus der Gruppe bestehend aus Morpholinyl, Thiomorpholinyl, Pyrrolidinonyl, Pyrrolidinyl, Piperidinyl, Homopiperidinyl, Piperazinyl, Homopiperazinyl, Hydantoinyl, Tetrahydropyridinyl, Tetrahydropyrimidinyl, Oxazolidinyl, Thiazolidinyl, Indolinyl, Isoindolinyl, Tetrahydrochinolinyl und Tetrahydroisochinolinyl.

5. Verbindung zur Verwendung nach einem der Ansprüche 1 bis 4, wobei R¹ entweder Aryl oder Heteroaryl ist.

6. Verbindung zur Verwendung nach einem der Ansprüche 1 bis 5, wobei R¹ ausgewählt ist aus der Gruppe bestehend aus Aryl, Furyl, Benzofuranyl, Thiophenyl, Benzothiophenyl, Chinolinyl, Pyrrolyl, Indolyl, Oxazolyl, Benzoxazolyl, Imidazolyl, Benzimidazolyl, Thiazolyl, Benzothiazolyl, Isoxazolyl, Pyrazolyl, Isothiazolyl, Pyridazinyl, Pyrimidinyl, Pyrazinyl, Triazinyl, Cinnolinyl, Phthalazinyl und Chinazolinyl.

7. Verbindung zur Verwendung nach einem der Ansprüche 1 bis 6, wobei R¹ Phenyl ist.

8. Verbindung zur Verwendung nach einem der Ansprüche 3 oder 4, wobei der Stickstoff-enthaltende Heterocyclus Piperazinyl, Piperidinyl oder Morpholinyl ist.

9. Verbindung A zur Verwendung nach Anspruch 1, genannt : 1-(4-{4-[4-(4-Chlor-Phenyl)-pyrimidin-2-ylamino]-benzoyl}-piperazin-1-yl)-ethanon und dargestellt durch die Strukturformel: 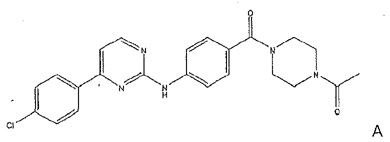 oder ein pharmazeutisch akzeptables Salz davon.

10. Verbindung zur Verwendung nach Ansprüchen 1 bis 9, wobei das multiple Myelom multiples Myelom Stadium I, multiples Myelom Stadium II, multiples Myelom Stadium III, asymptomatisches multiples Myelom, symptomatisches Myelom, vor kurzem diagnostiziertes multiples Myelom, responsives multiples Myelom, stabiles multiples Myelom, fortschreitendes multiples Myelom, rezidives multiples Myelom oder refraktäres multiples Myelom ist.

11. Verbindung zur Verwendung nach Anspruch 10, wobei das asymptomatische multiple Myelom schwelendes multiples Myelom oder indolentes multiples Myelom ist.

12. Pharmazeutische Zusammensetzung umfassend eine Verbindung gemäß Formel (I), (II), (III) oder A nach Ansprüchen 1 bis 9 oder ein physiologisch akzeptables Salz davon, und weiter umfassend eine effektive Menge mindestens eines Therapeutikums ausgewählt aus der Gruppe bestehend aus Bortezomib (Velcade), Melphalan, Prednison, Vincristin, Carmustin, Cyclophosphamid, Dexamathason, Thalidomid, Doxorubicin, Cisplatin, Etoposid und Cytarabin.

13. Pharmazeutische Zusammensetzung nach Anspruch 12, wobei das Therapeutikum Velcade oder Melphalan.

14. Verbindung zur Verwendung nach Ansprüchen 1 bis 9, zur Behandlung von multiplem Myelom in einem Individuum, das einer Strahlentherapie unterzogen wird, zur Vorbereitung für eine Stammzelltransplantation oder das einer Stammzelltransplantation unterzogen wird.

## Revendications

1. Composé de formule (I) : 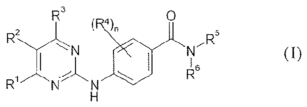 ou sel pharmacoloqiquement admissible d'un tel composé,
dans laquelle formule :
- R¹ représente un groupe aryle ou hétéroaryle, en option porteur d'un à quatre substituants choisi(s) dans l'ensemble de ceux qui sont symbolisés par R⁷ ;
- R² représente un atome d'hydrogène ;
- R³ représente un atome d'hydrogène ou un groupe alkyle inférieur ;
- R⁴ représente une entité choisie, indépendamment dans chaque cas, dans l'ensemble constitué par les atomes d'halogène et les groupes hydroxyle, alkyle inférieur et alcoxy inférieur ;
- l'indice n est un nombre entier valant de 0 à 4 ;
- R⁵ et R⁶ représentent des entités identiques ou différentes, choisies indépendamment dans l'ensemble de celles symbolisées par -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐNR⁹C(=O)R¹⁰, -(CH₂)ₐSO_{c}R⁹, ou -(CH₂)ₐSO₂NR⁹R¹⁰,
ou R⁵ et R⁶ représentent des entités qui, conjointement avec l'atome d'azote auquel elles sont liées, forment un hétérocycle, en option porteur d'un ou de substituant(s) ;
- R⁷ représente une entité choisie, indépendamment en chaque occurrence, dans l'ensemble formé par les atomes d'halogène, les groupes hydroxyle, cyano, nitro, carboxyle, alkyle, alcoxy, halogénoalkyle, acyloxy, sulfanyl-alkyle, sulfinyl-alkyle, sulfonyl-alkyle, hydroxyalkyle, aryle, aryle à substituant(s), alkyl-aryle, alkyl-aryle à substituant(s), hétérocycloalkyle, hétérocycloalkyle à substituant(s), alkyl-hétérocycloalkyle, et alkyl-hétérocycloalkyle à substituant(s), les groupes symbolisés par -C(=O)OR⁸, -OC(=O)R⁸, -C(=O)NR⁸R⁹, -C(=O)NR⁸OR⁹, -SO_{c}R⁸, -SO_{c}NR⁸R⁹, -NR⁸SO_{c}R⁹,-NR⁸R⁹, -NR⁸C(=O)R⁹, -NR⁸C(=O)(CH₂)_{b}OR⁹,-NR⁸C(=O)(CH₂)_{b}R⁹ ou -O(CH₂)_{b}NR⁸R⁹, et les groupes hétérocycloalkyle condensés avec un cycle phényle ;
- R⁸, R⁹, R¹⁰ et R¹¹ représentent des entités identiques ou différentes, qui sont choisies, indépendamment en chaque occurrence, dans l'ensemble constitué par un atome d'hydrogène et les groupes alkyle, alkyle à substituant(s), aryle, aryle à substituant(s), alkyl-aryle, alkyl-aryle à substituant(s), hétérocycloalkyle, hétérocycloalkyle à substituant(s), alkyl-hétérocycloalkyle et alkyl-hétérocycloalkyle à substituant(s), ou R⁸ et R⁹ représentent des entités qui, conjointement avec l'atome ou les atomes auquel ou auxquels elles sont liées, forment un hétérocycle, en option porteur d'un ou de substituant(s) ;
- les valeurs des indices a et b sont identiques ou différentes et sont choisies, indépendamment en chaque occurrence, dans l'ensemble formé par les nombres 0, 1, 2, 3 et 4 ;
- et l'indice c vaut, en chaque occurrence, 0, 1 ou 2 ;
pour utilisation dans le traitement d'un myélome multiple.

2. Composé pour utilisation, conforme à la revendication 1, choisi parmi ceux de formules (II), (III) et (IV) : 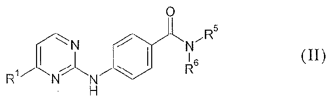 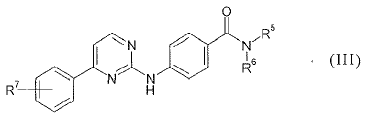 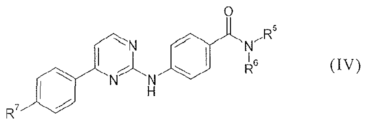 ou sel pharmacologiquement admissible d'un tel composé,
dans lesquelles formules R¹, R⁵, R⁶ et R⁷ ont les significations indiquées dans la revendication 1.

3. Composé pour utilisation, conforme à la revendication 1 ou 2, dans lequel R⁵ et R⁶ représentent des entités qui, conjointement avec l'atome d'azote auquel elles sont liées, forment un hétérocycle azoté non-aromatique, en option porteur d'un ou de substituant(s).

4. Composé pour utilisation, conforme à la revendication 3, dans lequel l'hétérocycle azoté non aromatique est un élément de l'ensemble constitué par les groupes morpholinyle, thiomorpholinyle, pyrrolidinonyle, pyrrolidinyle, pipéridinyle, homopipéridinyle, pipérazinyle, homopipérazinyle, hydantoïnyle, tétrahydropyridinyle, tétrahydropyrimidinyle, oxazolidinyle, thiazolidinyle, indolinyle, isoindolinyle, tétrahydroquinolyle et tétrahydroisoquinolyle.

5. Composé pour utilisation, conforme à l'une des revendications 1 à 4, dans lequel R¹ représente un groupe aryle ou hétéroaryle.

6. Composé pour utilisation, conforme à l'une des revendications 1 à 5, dans lequel R¹ représente un élément de l'ensemble constitué par les groupes aryle, furyle, benzofuryle, thiényle, benzothiényle, quinolyle, pyrrolyle, indolyle, oxazolyle, benzoxazolyle, imidazolyle, benzimidazolyle, thiazolyle, benzothiazolyle, isoxazolyle, pyrazolyle, isothiazolyle, pyridazinyle, pyrimidinyle, pyrazinyle, triazinyle, cinnolinyle, phtalazinyle et quinazolinyle.

7. Composé pour utilisation, conforme à l'une des revendications 1 à 6, dans lequel R¹ représente un groupe phényle.

8. Composé pour utilisation, conforme à la revendication 3 ou 4, dans lequel l'hétérocycle azoté est un groupe pipérazinyle, pipéridinyle ou morpholinyle.

9. Composé A pour utilisation, conforme à la revendication 1, dont le nom est : 1-(4-{4-[4-(4-chloro-phényl)-pyrimidin-2-yl-amino]-benzoyl}-pipérazin-1-yl)-éthanone, et représenté par la formule structurale suivante : 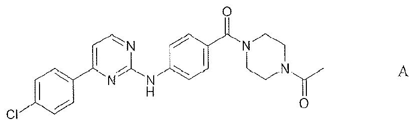 ou sel pharmacoloqiquemcnt admissible de ce composé.

10. Composé pour utilisation, conforme à l'une des revendications 1 à 9, le myélome multiple étant un myélome multiple de stade I, un myélome multiple de stade II, un myélome multiple de stade III, un myélome multiple asymptomatique, un myélome symptomatique, un myélome multiple de diagnostic récent, un myélome multiple sensible au traitement, un myélome multiple stable, un myélome multiple en évolution, un myélome multiple récidivant, ou un myélome multiple réfractaire au traitement.

11. Composé pour utilisation, conforme à la revendication 10, le myélome multiple asymptomatique étant un myélome multiple en phase d'incubation ou un myélome multiple peu évolutif.

12. Composition pharmaceutique comprenant un composé de formule (I), (II), (III) ou A, conforme à l'une des revendications 1 à 9, ou un sel physiologiquement admissible d'un tel composé, et comprenant en outre, en quantité efficace, au moins un agent thérapeutique choisi dans l'ensemble formé par les suivants : bortézomib (velcade), melphalan, prednisone, vincristine, carmustine, cyclophosphamide, dexaméthasone, thalidomide, doxorubicine, cisplatine, étoposide et cytarabine.

13. Composition pharmaceutique conforme à la revendication 12, pour laquelle l'agent thérapeutique est choisi parmi le velcade et le melphatan.

14. Composé pour utilisation, conforme à l'une des revendications 1 à 9, destiné au traitement d'un myélome multiple chez un sujet en train de subir une radiothérapie, en cours de préparation en vue d'une transplantation de cellules souches, ou en train de subir une transplantation de cellules souches.
